# EUROPEAN PATENT APPLICATION

(11) **EP 2 181 708 A1**
(43) Date of publication of application: **05.05.2010**
(21) Application number: 08382062.1
(22) Date of filing: 31.10.2008
(51) Int. Cl.: A61K 31/19, A61P 25/00

(54) **Use of Valproate for reducing CHOP levels, particularly in a mammal having SCI**

(71) Applicant: Universitat Autonoma de Barcelona, 08193 Bellaterra (ES)
(72) Inventor: Casas Louzao, Catalina, 08193, BELLATERRA (ES); Penas Pérez, Clara, 08193, BELLATERRA (ES); Navarro Acebes, Xavier, 08193, BELLATERRA (ES); Verdu Navarro, Enric, 08193, BELLATERRA (ES)
(74) Representative: Ponti Sales, Adelaida

(57) **Abstract**

The present invention relates to the use of valproate in the manufacture of a drug for reducing CHOP levels in a mammal. In particular, said mammal has spinal cord injury (SCI), particularly traumatic SCI. The present invention, therefore, also relates to the use of valproate in the manufacture of a drug for treating SCI in a mammal, particularly traumatic SCI. The present invention also relates to a method for reducing CHOP levels in a mammal, particularly said mammal having spinal cord injury (SCI), specifically traumatic SCI. The present invention also relates to valproate for use in reducing CHOP levels in a mammal, particularly said mammal having spinal cord injury (SCI), specifically traumatic SCI.

## Description

### Field of invention

The present invention relates to the use of valproate in the manufacture of a drug for reducing CHOP levels in a mammal. In particular, said mammal has spinal cord injury (SCI), particularly traumatic SCI. The present invention, therefore, also relates to the use of valproate in the manufacture of a drug for treating SCI in a mammal, particularly traumatic SCI. The present invention also relates to a method for reducing CHOP levels in a mammal, particularly said mammal having spinal cord injury (SCI), specifically traumatic SCI. The present invention also relates to valproate for use in reducing CHOP levels in a mammal, particularly said mammal having spinal cord injury (SCI), specifically traumatic SCI.

### Background of invention

Spinal cord injury represents a severe health problem associated with life-long disabilities. The effects of SCI can be devastating. The injury usually results in permanent paralysis of voluntary muscles and loss of sensation below the level of the lesion, which is associated with reduced mobility and functional independence, impairment of social and vocational activities, as well as negative influences on the person's health and well-being. There are no fully restorative therapies for SCI as yet and so prevention (for example, effective seat belts, weapons restrictions and safety in sports) is the best medicine. SCI has a significant impact on quality of life, life expectancy and economic burden, with considerable costs associated with primary care and loss of income. Quadriplegics ranked recovery of arm and hand function as a priority, whereas paraplegics rated recovery of sexual function as most important (when measured against recovery of bladder/bowel function, and eradicating autonomic dysreflexia, improving walking movements and trunk stability, regaining normal sensation and eliminating chronic pain). Thus recovery of limb function is the focus of most ongoing animal studies and clinical trials for treatments for SCI.

From a biological point of view, SCI, namely traumatic SCI, evolves through three phases: the acute, mostly due to the direct mechanical damage, the secondary and the chronic phases (Tator and Fehlings, 1991). Besides the immediate cell death occurring after SCI, apoptosis contributes to a delayed, prolonged death of neurons and oligodendrocytes, causing progressive degeneration of the spinal cord (Emery et al., 1998); (Penas et al., 2007). Many apoptotic oligodendrocytes are found in the white matter along fiber tracts undergoing Wallerian degeneration after SCI, and this apoptosis ultimately contributes to chronic demyelination and spinal cord dysfunction (Beattie et al., 2002). At the site of the lesion, there is loss of segmental neurons(Garcia-Alias et al., 2006); (Collazos-Castro et al., 2005), producing, among other effects, denervation atrophy of the segmental muscles affected. Several studies have pointed out the importance of neuroprotective therapeutical approaches to reduce the loss of spinal motoneurons and oligodendrocytes after injury (Tamaki et al., 2008).

The present inventors have recently reported (Penas et al., 2007) that SCI induces endoplasmic reticulum (ER) stress revealed by the activation of an unbalanced unfolded protein response (UPR). UPR is characterized by the activation of concurrent pathways that will finally allow the cell to recover from the stress or fatally die by apoptosis (Mamady and Storey, 2008). The balance between two different proteins, the chaperone Bip/Grp78 and the C/EBP homologous transcription factor (CHOP) drives the cellular destiny. Bip/Grp78 is a key factor triggering the UPR and is considered a pro-survival molecule (Wang et al., 1996) in charge of the correct folding of accumulated proteins (Bertolotti et al., 2000); whereas CHOP is considered an ER stress-inducer of apoptosis (Zinszner et al., 1998); (Oyadomari et al., 2002) (Oyadomari et al., 2001). The present inventors found that the UPR triggered after SCI is unbalanced towards apoptosis in neurons and oligodendrocytes, as evidenced by progressive nuclear accumulation of CHOP and unchanged levels of BiP/Grp78 (Penas et al., 2007). Furthermore, the astrocytes which are capable of survive and even proliferate after SCI did not present this nuclearization of CHOP. That is, CHOP marks precisely the cells prone to die after SCI. Besides, the present inventors have also observed as shown herein that the levels of CHOP are directly proportional with the severity degree of the lesion; i.e. the more severe, the higher its levels. Considering that Grp78 overexpression may prevent the apoptosis promoted by CHOP after ER stress (Wang et al., 1996), the present inventors investigated therapeutical strategies to increase the levels of this chaperone to promote neuroprotection after SCI.

So far, the therapeutical approaches for SCI is limited to the early administration of methylprednisolone sodium succinate (MPSS), an steroid which has been the most extensively studied compound among other neuroprotective agents for SCI. MPSS acts mainly by stabilising phospholipid-membrane structures and by reducing the inflammatory response. In a series of clinical studies, the National Acute Spinal Cord Injury Studies (NASCIS I-III), MPSS was given alone or in combination with either the opioid antagonist naloxone or the lipid peroxidation inhibitor, tirilazad mesylate. In addition, based on beneficial effects in animal experiments, clinical studies have been carried out with GM-1-ganglioside, thyrotropin releasing hormone (TRH), the calcium channel blocker Nimodipine and the NMDA-receptor antagonist Gacyclidine. Although none of these studies has shown significant beneficial clinical effects, administration of MPSS within 8 hours after injury is today considered as a "treatment option". Nevertheless if any benefit is produced by MPSS, it is probably small and has not been demonstrated by the NASCIS trials. Even assuming that MPSS has positive effects on the outcome of SCI, both the risks to the patient and the cost of the additional resources required to treat the complications should be outweighed by its therapeutic benefits. High steroid levels may produce acute corticosteroid myopathy, which may take months to resolve, and indeed, this resolution may account in part for whatever improvement in motor strength has been attributed to the treatment.

The compound used in the present invention, valproate (VPA), is a multitarget drug that it has been shown to act either as a neuroprotector on brain ischemia animal models and as an apoptotic inducer in tumor cells, effects that depend on the concentrations used and duration of the treatment. For this reason, VPA neuroprotective effects after SCI were not evident. The drug is currently administered for the treatment of epilepsy and mania in patients with mood disorders. VPA is also used for acute and maintenance therapy of bipolar disease, for migraine prophylaxis, and occasionally for chronic pain syndromes. In the present invention, when analysing the effect of VPA treatment on the Grp78/CHOP ratio after SCI as well as its neuroprotective potential, the present inventors have surprisingly found that VPA reduces demyelination and cavity volume, protects neurons and axons and promote functional recovery after SCI. To be highlighted, MPSS has been described to promote the same effects with the exception that it does not sustain neuronal survival at the damaged spinal cord. Thus, a better tissue recovery is achieved with VPA than with MPSS in animal models which renders VPA a more suitable therapeutic agent with better clinical outcomes for treating SCI than MPSS.

### Summary of the invention

The present invention relates to the use to the use of valproate in the manufacture of a drug for reducing CHOP levels in a mammal. In particular, said mammal has spinal cord injury (SCI), particularly traumatic SCI. The present invention, therefore, also relates to the use of valproate in the manufacture of a drug for treating SCI in a mammal, particularly traumatic SCI. The present invention also relates to a method for reducing CHOP levels in a mammal, particularly said mammal having spinal cord injury (SCI), specifically traumatic SCI. The present invention also relates to valproate for use in reducing CHOP levels in a mammal, particularly said mammal having spinal cord injury (SCI), specifically traumatic SCI.

### Brief description of drawings

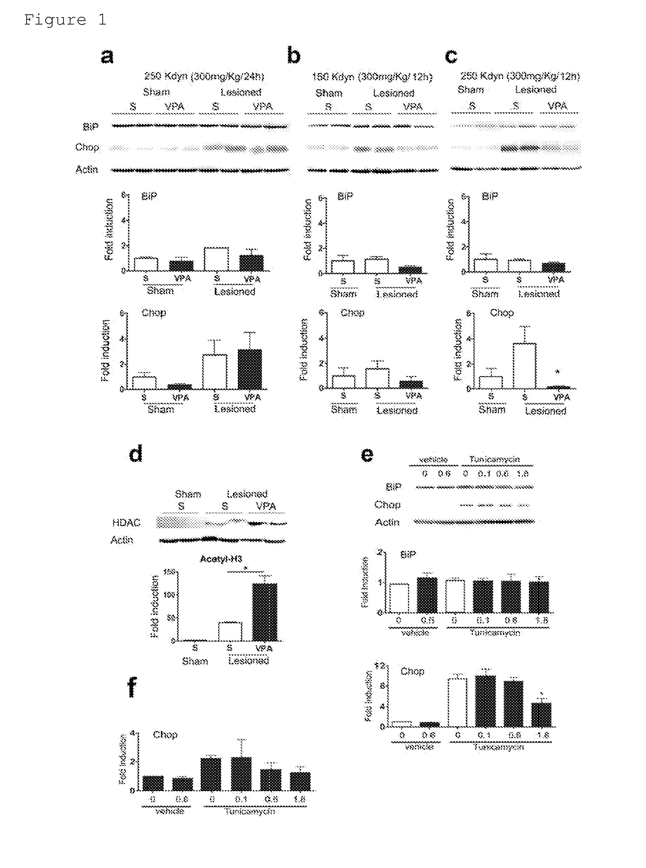

**Figure 1** VPA treatment reduces CHOP accumulation after ER stress and SCI. **(a-c)** Effects of VPA administration to a rat model of SCI on injured-induced CHOP accumulation over sham spinal cord in comparison with vehicle-saline administration. Each blot corresponds to representative samples (20 µg total protein loaded) obtained from animals submitted to different contusion severity (150 or 250 Kdyn) and/or different regimes of valproate administration (every 12 or 24h). Western blot analysis shows an increase of CHOP proportional to the severity of the damage while no-changes of BiP relative levels. Histograms below represent the average ± SEM of BiP and CHOP protein levels relative to actin levels in the spinal cord of sham and lesioned animals either injected with saline (S) or valproate (VPA) during three days post-injury. (n=5 rats per group).**P* < 0.05. **(d)** Western blot analysis shows the increase of acetylated histone 3 (acetyl-H3) protein levels relative to the actin in animals submitted to 250 Kdyn contusion and treated with VPA every 12 h. (n=5 rats per group)* *P* < 0.05.**(e)** Effects of VPA at different concentrations (0-1.8 nM) on accumulative CHOP after ER stress-induction using 3 µg/ml tuncamycin on fibroblast cells (n=4)* *P* < 0.05 All statistical data are obtained by one-way ANOVA followed by Bonferroni's multiple comparision test. **(f)** The same analysis as in (e) with HEK cell line.

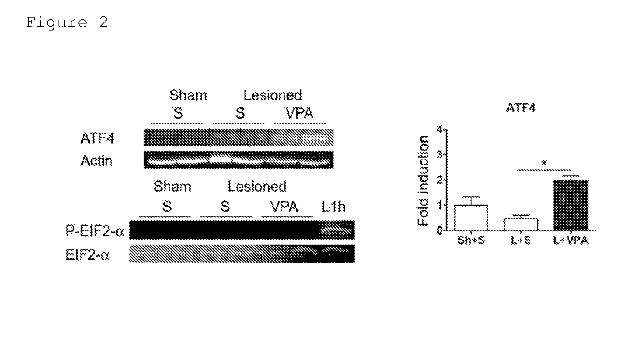

**Figure 2** Effects of VPA treatment on other UPR-related proteins. Western blot analysis and bar histogram representing the average ± SEM of ATF4 levels relative to actin, and of phospho-eIF2α protein (P-eIF2α) levels with respect to eIF2α levels in the spinal cord of sham (Sh) and lesioned (L) animals treated with saline (S) or valproate (VPA). Spinal cord tissue remove 1h post-injury was used as a control on P-eIF2α analysis (100 µg total protein loaded). * *P* < 0.01, one-way ANOVA followed by Bonferroni's multiple comparison test.

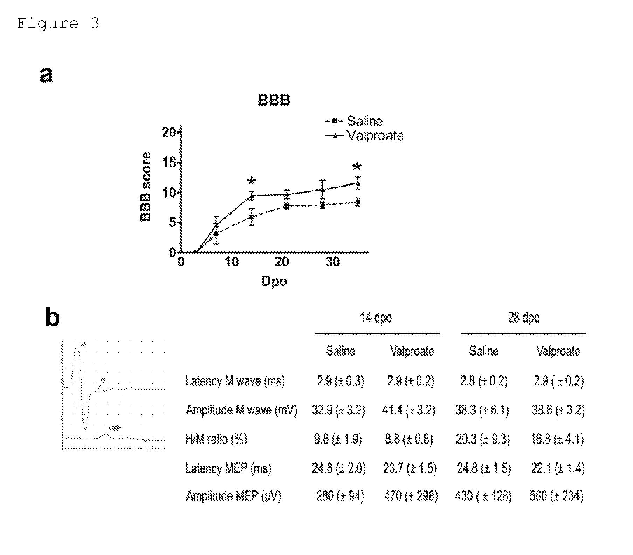

**Figure 3** Effect of VPA on the recovery of coordinated hind limb locomotion. **(a)** Evolution of the BBB score in the open field walking test for the groups of rats subjected to SCI (250 Kdyn impact) and saline or VPA administration (300 mg/kg every 12 h during one week and every 24 h during two more weeks) and followed for 35 days after the lesion. Data points represent the average ± SEM. BBB scale range from 21 in normal to 0 in rats with complete hind limb paralysis * *P* < 0.05, unpaired t-test in each time analysed. **(b)** Representative recording and electrophysiological results of nerve conduction tests and motor evoked potentials (MEP) in tibialis anterior muscles at 14 and 28 days after spinal cord contusion (SCI; 250 Kdyn) of both saline and valproate treated SCI-rats.

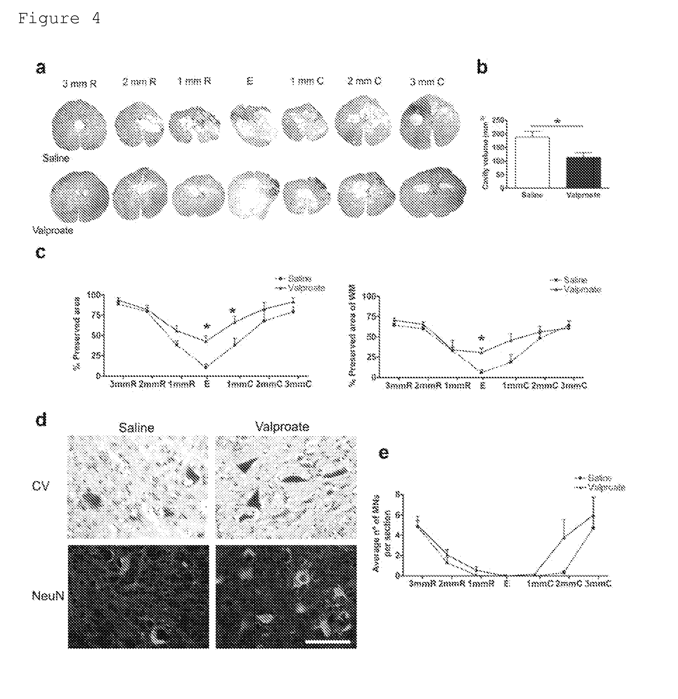

**Figure 4** VPA improves spinal cord tissue sparing and neuronal survival after SCI.
   **(a)** Representative transverse spinal cord Klüber-Barrier stained sections at 35 days after T8 contusion injury at the epicenter (E) and at 1 mm increments rostral (R) and caudally (C) in a rat receiving saline vehicle (top) and valproate (bottom). **(b)** Histogram showing the estimated volume of the spinal cord lesion in both groups (* *P* < 0.05 Unpaired t test). **(c)** Measurements of the area of preserved cord tissue (left graph) and of white matter (right graph) at the injury epicenter and adjacent tissue up to 3 mm rostral and caudally (* *P* < 0.05 two-way,ANOVA followed by Bonferroni post-hoc test). **(d)** Representative microphotographs of the anterior ventral horns showing motoneurons stained with cresyl violet (CV) or immunostained with NeuN antibody at 2 mm caudal to the lesion epicentre. **(e)** Bar histogram representing the average number of motoneurons per section. Scale bar, 100 µM. (n= 5 per group)

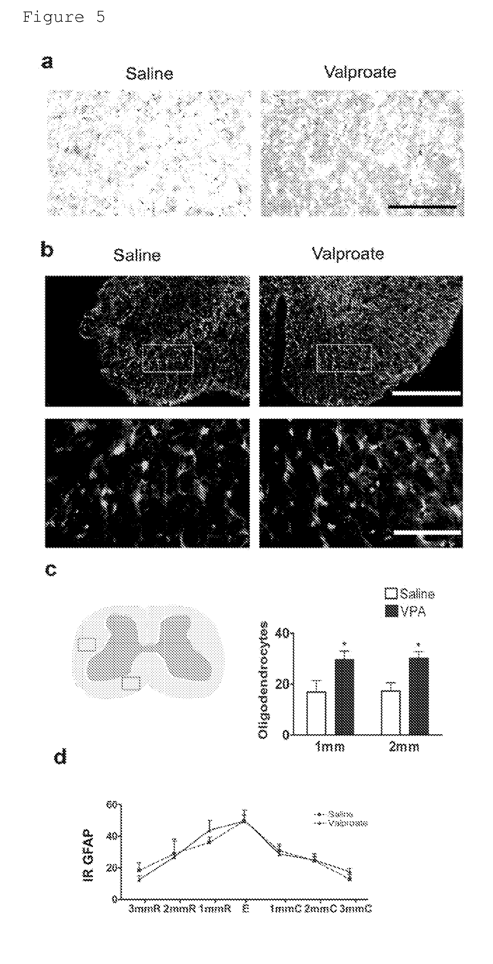

**Figure 5** VPA effects on glia. **(a)** High resolution microphotographs of equivalent white matter areas of the anterior funiculus in cresyl violet and luxol fast blue stained spinal cord sections from saline or VPA-treated SCI-rats at 35 dpo showed that VPA treatment reduces myelin loss. Transverse cryosections were selected 2 mm caudal to the lesion epicenter. Scale bar, 100 µM. **(b)** Representative microphotographies showing different APC-positive oligodendrocyte quantity in the same region, 2 mm distal to the epicenter. Boxed areas are magnified in the panels at the bottom. Scale bars, 400 µM (top), 100 µM (bottom). **(c)** VPA treatment reduces oligodendrocyte number loss at 1 and 2 mm caudal to the lesion epicenter at both the dorsal and anterior funiculum (boxed areas in the spinal cord diagram).( ( n= 5 rats per group) * *P* < 0.05, Two-way ANOVA followed by Bonferroni post-hoc test. **(d)** Overall astroglial GFAP-positive immunoreactivity (IR) was not altered by the treatment at 35 dpo.

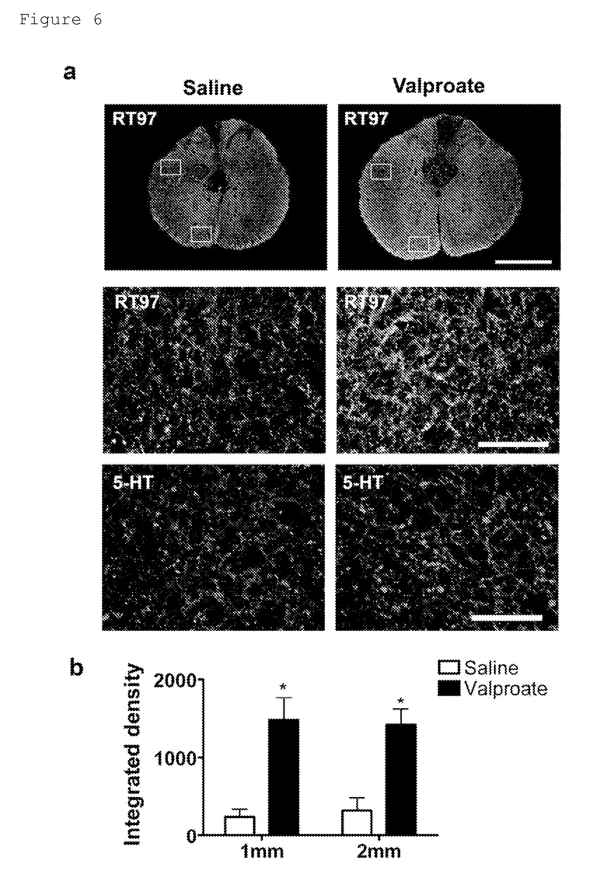

**Figure 6** VPA treatment reduces axonal loss following SCI. **(a)** Microphotographs of spinal cord sections at 2 mm distal to the epicenter immunostained against heavy neurofilament (RT97) or serotonin (5-HT). Inferior panels are representative magnified microphotographs of the area from the anterior funiculum. Scale bar, 800 µM (top), 100 µM. **(b)** VPA treatment increases the NF immunoreactivity analyzed by fluorescent densitometry obtained from the boxed areas indicated in (a) at 2 and 3 mm. **P* < 0.01.

### Description of the invention

The present invention relates to the use of valproate in the manufacture of a drug for reducing CHOP levels in a mammal. In particular, said mammal has spinal cord injury (SCI), particularly traumatic SCI.

The present invention, therefore, also relates to the use of valproate in the manufacture of a drug for treating SCI in a mammal, particularly traumatic SCI.

The present invention also relates to a method for reducing CHOP levels in a mammal, particularly said mammal having spinal cord injury (SCI), preferably traumatic SCI.

The present invention also relates to valproate for use in reducing CHOP levels in a mammal, particularly said mammal having spinal cord injury (SCI), preferably traumatic SCI.

Valproate refers to the ionic form of valproic acid (2-propylpentanoic acid) in water. Valproic acid has the following structure:

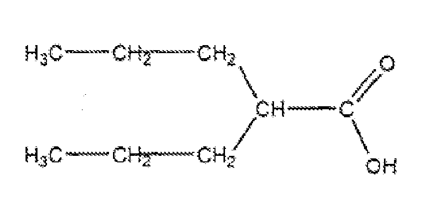

Indeed, the valproate ion is absorbed and produces the therapeutic effect. As such, any reference to "valproate" or "valproate compound" should be construed as including a compound which disassociates within the gastrointestinal tract, or within *in vitro* dissolution media, to produce a valproate ion. As such, valproate includes but is not limited to, valproic acid, any of the various salts of valproic acid described below, any of the prodrugs of valproic acid described below, any hydrated form of the aforementioned compounds, as well as any combination thereof.

Valproic acid is available commercially from Abbott Laboratories of Abbott Park, Ill., USA. Methods for its synthesis are described in Oberreit, Ber. 29, 1998 (1896) and Keil, Z. Physiol. Chem. 282, 137 (1947), the contents of both which are hereby incorporated by reference.

Suitable pharmaceutically acceptable basic addition salts of valproic acid include, but are not limited to cations based on alkali metals or alkaline earth metals such as lithium, sodium, potassium, calcium, magnesium and aluminium salts and the like, and non-toxic quaternary ammonia and amine cations including ammonium, tetramethylammonium, tetraethylammonium, methylamine, dimethylamine, trimethylamine, triethylamine, diethylamine, ethylamine, and the like. Other representative organic amines useful for the formation of base addition salts include ethylenediamine, ethanolamine, diethanolamine, piperidine, piperazine and the like.

Valproate sodium is a popular salt of valproic acid. Methods for the preparation of valproate sodium may be found in US4,988,731 and US5,212,326, the contents of which are hereby incorporated by reference. Like valproic acid, valproate sodium also disassociates within the gastrointestinal tract to form a valproate ion.

Compounds comprising combinations of sodium valproate and valproic acid (divalproex sodium, for example) are bioavailable and therapeutically active sources of valproate. The non-stoichiometric compound known as divalproex sodium is disclosed in US4,988,731.

In addition to the above-mentioned compounds, one of ordinary skill in the art would readily recognize that the carboxylic moiety of the valproate compound may be functionalized in a variety of ways. This includes forming compounds which readily metabolize *in vivo* to produce valproate, such as valproate amide (valproimide), as well as other pharmaceutically acceptable amides and esters of the acid (i.e. prodrugs).

"Pharmaceutically acceptable ester" refers to those esters which retain, upon hydrolysis of the ester bond, the biological effectiveness and properties of the carboxylic acid and are not biologically or otherwise undesirable. For a description of pharmaceutically acceptable esters as prodrugs, see Bundgaard, E., ed., (1985) Design of Prodrugs, Elsevier Science Publishers, Amsterdam. These esters are typically formed from the corresponding carboxylic acid and an alcohol. Generally, ester formation can be accomplished via conventional synthetic techniques (see, e.g., March Advanced Organic Chemistry, 3rd Ed., John Wiley & Sons, New York p. 1157 (1985) and references cited therein, and Mark et al. Encyclopedia of Chemical Technology, John Wiley & Sons, New York (1980)). The alcohol component of the ester will generally comprise (i) a C₂-₁₂aliphatic straight or branched alcohol optionally with one, two, or three double bonds; or (ii) a C₇-₁₂ aromatic or heteroaromatic alcohol. The present invention also contemplates the use of those compositions which are esters as described herein and at the same time are the pharmaceutically acceptable salts thereof.

"Pharmaceutically acceptable amide" refers to those amides which retain, upon hydrolysis of the amide bond, the biological effectiveness and properties of the carboxylic acid and are not biologically or otherwise undesirable. For a description of pharmaceutically acceptable amides as prodrugs, see Bundgaard, H., Ed., (1985) Design of Prodrugs, Elsevier Science Publishers, Amsterdam. These amides are typically formed from the corresponding carboxylic acid and an amine. Generally, amide formation can be accomplished via conventional synthetic techniques (see, e.g., March Advanced Organic Chemistry, 3rd Ed., John Wiley & Sons, New York, p. 1152 (1985) and Mark et al. Encyclopedia of Chemical Technology, John Wiley & Sons, New York (1980)). The present invention also contemplates the use of those compositions which are amides, as described herein, and at the same time are the pharmaceutically acceptable salts thereof.

The term "spinal cord injury" or also termed SCI is a well-characterized pathology and is used herein as already expressed herein in the background section i.e. as an insult to the spinal cord resulting in a change, either temporary or permanent, in its normal motor, sensory, or autonomic function, whose origin can be a trauma (falls, car crashes, sport injuries, gun shots, diving accidents, war injuries, violent attacks, or any event where the spinal cord may be affected by a direct impact on spinal cord) or a disease (Transverse Myelitis, Polio, Spina Bifida, Friedreich's Ataxia, etc.).

In a preferred embodiment, the present invention is addressed to traumatic SCI i.e caused by any of trauma mentioned above and according to the American Spinal Injury Association (ASIA) and the International Spinal Cord Injury Classification System can be cassified into five types
- "A" indicates a "complete" spinal cord injury where no motor or sensory function is preserved in the sacral segments S4-S5. Since the S4-S5 segment is the lower segmental, absence of motor and sensory function indicates "complete" spinal cord injury.
- "B" indicates an "incomplete" spinal cord injury where sensory but not motor function is preserved below the neurological level and includes the sacral segments S4-S5. This is typically a transient phase and if the person recovers any motor function below the neurological level, that person essentially becomes a motor incomplete, i.e. ASIA C or D.
- "C" indicates an "incomplete" spinal cord injury where motor function is preserved below the neurological level and more than half of key muscles below the neurological level have a muscle grade of less than 3.
- "D" indicates an "incomplete" spinal cord injury where motor function is preserved below the neurological level and at least half of the key muscles below the neurological level have a muscle grade of 3 or more.
- "E" indicates "normal" where motor and sensory scores are normal. Note that it is possible to have spinal cord injury and neurological deficit with completely normal motor and sensory scores.

When using "traumatic SCI" herein it relates interchangeably to any of these stages of a traumatic SCI.

The terms "treatment" or "treating" a spinal cord injury refers to, inter alia, reducing or alleviating one or more symptoms in a subject, as well as slowing or reducing progression of existing disease. For a given subject, improvement in a symptom, its worsening, regression, or progression may be determined by an objective or subjective measure. Efficacy of treatment may be measured as improvement of motor and sensory functions, level of the functional deficits, improvement of pain symptoms, as the main primary outcome measures.

In another embodiment, the present invention relates to the aforementioned use of valproate wherein the subject to be treated is particularly a mammal, especially human beings and rodent or primate models of disease. Thus, both veterinary and medical methods are contemplated.

In a further embodiment, the present invention concerns the use of valproate for the manufacture of a drug for reducing CHOP levels, particularly in a subject having SCI and, preferably, said SCI being traumatic SCI, wherein valproate is formulated in a pharmaceutical composition comprising a therapeutically effective amount of valproate, and a pharmaceutically acceptable carrier. A "therapeutically effective amount" in this context is an amount sufficient to act against, to stabilize or to reduce the symptoms caused by a spinal cord injury in a mammal and administered according to the dosage regimen disclosed below. In a further embodiment, valproate is used as a neuroprotective agent in a mammal having traumatic SCI.

Therefore, valproate may be formulated into various pharmaceutical forms for administration purposes. As appropriate compositions there may be cited all compositions usually employed for systemically administering drugs. To prepare the pharmaceutical compositions of this invention, an effective amount of valproate as the active ingredient is combined in intimate admixture with a pharmaceutically acceptable carrier, which carrier may take a wide variety of forms depending on the manner of preparation desired for administration. These pharmaceutical compositions are desirable in unitary dosage form suitable, particularly, for administration orally, rectally, transdermally, by inhalation, or by parenteral routes (i.e., subcutaneous, intravenous, intramuscular or intraperitoneal). Valproate can be administered by the oral route in solid dosage forms, such as tablets, capsules, and powders, or in liquid dosage forms, such as elixirs, syrups, and suspensions. The pharmaceutical compositions of this invention can also be administered parenterally, in sterile liquid dosage forms.

In preparing the compositions in oral dosage form, any of the usual pharmaceutical media may be employed such as, for example, water, glycols, oils, alcohols and the like in the case of oral liquid preparations such as suspensions, syrups, elixirs, emulsions and solutions; or solid carriers such as starches, sugars, kaolin, lubricants, binders, disintegrating agents and the like in the case of powders, pills, capsules, and tablets. Because of their ease in administration, tablets and capsules represent the most advantageous oral dosage unit forms, in which case solid pharmaceutical carriers are obviously employed.

Valproate may as well be administered in controlled, sustained or slow release oral dosage forms such as the ones described in patents US2005276850, EP1219295, EP1216704, or US5589191.

Formulations for parenteral administration may be in the form of aqueous or non-aqueous isotonic sterile injection solutions or suspensions. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations which are intended to be converted, shortly before use, to liquid form preparations. In the compositions suitable for percutaneous administration, the carrier optionally comprises a penetration enhancing agent and/or a suitable wetting agent, optionally combined with suitable additives of any nature in minor proportions, which additives do not introduce a significant deleterious effect on the skin. These latter suitable additives may be anti-oxidants, preservatives, stabilizing agents, emulsifiers, salts for influencing the osmotic pressure, and/or buffer substances.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, suppositories, powder packets, wafers, injectable solutions or suspensions and the like, and segregated multiples thereof.

In a further embodiment, the invention relates to a dosage regimen of valproate in its use for the manufacture of a drug for reducing CHOP levels, particularly in a subject having SCI and, preferably, said SCI being traumatic SCI. A daily oral dosage for treating SCI, in particular traumatic SCI, comprises the initial administration of approximately from 1 to 15 mg/kg total body weight of valproate after at least 3 hours post-injury and a daily administration of approximately from 20 to 60 mg/kg, preferably approximately from 30 to 50 mg/kg total body weight of valproate in adult subjects from 12 hours post-injury. The duration of the treatment must last at least 21 days post-injury. In other words, the treatment can be divided in two steps:
- immediately after injury occurs no drug must be administered since the system must reach an stabilization and bleeding must be over. Therefore, the treatment should start only 3 hours post-injury;
- at 12 hours from the first doses post-injury, it is already possible to administer a regular daily oral dose in a regime from 8 to 12 hours. This treatment should be continued until at least 21 days post-injury.

Valproate may be used as a single therapeutic agent or in combination with other therapeutic agents. When administered as a combination, the therapeutic agents can be formulated as separate compositions that are administered simultaneously or sequentially at different times, or the therapeutic agents can be given as a single composition.

Therapeutical approaches for SCI include the use of multitarget neuroprotective agents, which might preserve as much tissue as possible, suppress some of the damaging mechanisms implicated in the secondary lesion expansion and improve long tracts integrity, that in combination with proregenerative treatments would eventually allow for reconnection of disrupted spinal tracts and functional recovery.

The co-therapy or combination-therapy, in defining the use of valproate and another pharmaceutical agent, is intended to embrace administration of each drug in a sequential manner in a regimen that will provide beneficial effects of the drug combination, and is intended as well to embrace co-administration of these drugs in a substantially simultaneous manner, such as in a single dosage form having a fixed ratio of these active drugs or in multiple, separate dosage forms for each drug. Specifically, the administration of valproate can be carried out in conjunction with additional therapies known to those skilled in the art in the treatment of SCI, such as other active ingredients, stem cells or other cell types selected from the group comprising Schwann cells and olfactory ensheating glial cells.

Accordingly, the present invention relates to the use of valproate for the manufacture of a drug useful for treating spinal cord injury in a mammal wherein said drug is used in a combination therapy, said combination therapy preferably comprising valproate and another pharmaceutical agent.

The present invention is illustrated by the following examples, which are not to be construed as limiting.

In order to assess the results obtained with the administration of valproate, several functional recovering parameters of locomotive standards have been analysed.

### Example

### MATERIALS AND METHODS

### Surgical procedure

Adult females Sprague-Dawley rats aged 11 weeks (220-250 g) were anesthetized with pentobarbital (40 mg/kg i.p.). When unresponsive, the vertebral column was exposed and a laminectomy carried out at T8-T9 levels. Rostral T7 and caudal T10 vertebral bodies were clamped with Adson stabilizing forceps, and carefully adjusted to level the spinal cord in the horizontal plane. A contusion injury was performed using the Infinite Horizon impactor device (Precision Scientific Instruments, Lexington, KY). The stainless steel impounder tip was placed at 3-4 mm above the exposed spinal cord, and then a fast impact produced applying forces of 150 or 250 kilodynes (KDyn) to the exposed spinal cord. The wound was sutured with 5-0 silk thread in the muscular plane and the skin was closed with small clips and disinfected with povidone iodine solution. Sham-operated animals received the same surgical procedure but sustained no impact injury; the spinal cord was left exposed for 5 min. The animals were maintained in a warm environment until full recovery, and received preventive antibiotic (amoxicyline) and analgesic (buprenorphine) treatment. All animals were kept under standard conditions of light and temperature and fed with food and water ad libitum. The procedures involving animals were approved by the Ethics Committee of our institution, and followed the European Communities Council Directive 86/609/EEC.

### Valproate administration

VPA was intraperitoneally administered at an initial dose of 150 mg/kg by 3 h after surgery, and then a dose of 300 mg/Kg either every 24 h or every 12 h during the first week post-surgery, following by a dose of 300 mg/Kg every 24 h for the next two weeks. Groups of rats subjected to SCI or sham operation received VPA or saline injections following the same regime. Experiments were approved by the Ethics Committee at the Universitat Autònoma de Barcelona, and followed the European Communities Council Directive 86/609/EEC

### Functional and electrophysiological assessment

Locomotor activity was evaluated before surgery and at 3, 7, 14 and 28 days post-lesion, using the open-field walking scoring system that measures locomotor ability during 5 min. One animal at a time was allowed to move inside a circular enclosure (90 cm diameter x 24 cm wall height). Two independent examiners observed the hindlimbs movements and scored the locomotor function according to the Basso, Beattie and Bresnahan (BBB) scale (Basso et al., 1996). Briefly, the BBB testing scale consists of an ordinal scale from 0 points (no discernable hind limb movement) to 21 points (consistent, coordinated gait with parallel paw placement of the hind limb and consistent trunk stability). The final score of each animal was obtained by averaging the values from both examiners.

Electrophysiological tests were performed at 14 and 28 days after operation. Animals were anesthetized with sodium pentobarbital (30 mg/kg, i.p.), and placed in a prone position over a warmed flat coil to maintain body temperature. For assessment of peripheral nerve conduction and spinal reflexes, the sciatic nerve was stimulated with single electrical pulses of 0.1 ms duration up to supramaximal intensity delivered by monopolar needles inserted percutaneously at the sciatic notch. Compound muscle action potentials, including the direct muscle response (M wave) and the monosynaptic reflex response (H wave), of the tibialis anterior muscle were recorded by mean of needle electrodes, amplified and displayed on the oscilloscope to measure the amplitude of the negative peak and the latency of M and H waves (Valero-Cabre et al., 2004). The H/M amplitude ratio was calculated. Motor evoked potentials (MEPs) were elicited by transcranial stimulation with two needle electrodes placed subcutaneously over the skull, delivering pulses of 0.1 ms duration and 25 mA intensity, and recorded from the tibialis anterior muscle (Garcia-Alias et al., 2003). The signals were amplified, filtered and displayed on the oscilloscope (Sapphire 4ME, Oxford Instruments, Surrey, UK) to measure the amplitude and the latency of the evoked potentials.

### Tissue processing for histology and immunohistochemistry

At 3 or 35 days post-surgery, rats were perfused transcardially with 4% paraformaldehyde in 0.1M phosphate buffer saline (PBS, pH 7.4). Spinal cord blocks containing T6-T10 spinal cord segments were removed, post-fixed overnight in the same fixative solution, and cryoprotected in 30% sucrose at 4°C. Two spinal cord blocks, one of a saline-treated rat and the other of a valproate-treated rat were pairly embedded in TissueTek (Sakumura, Netherlands) and serially cut (20 µm thickness) in the transverse plane in a cryostat. Seven series of 10 slides containing 5 sections of each spinal cord were collected onto gelatin-coated glass slides, so that each serie comprehend 1 mm length of the spinal cord.

### Cell culture

Primary fibroblasts obtained from rat sciatic nerve and HEK cell line were cultured in Dulbecco's modified Eagle's/F-12 medium supplemented with 10% fetal calf serum (Gibco). Fibroblasts were treated with 3 µg/mL of tunicamycin (Sigma) for 4 h and harvested in RIPA buffer for protein extraction.

### Histological analysis

For each pair of spinal cords, one slide of each mm was stained with luxol fast blue and cresyl violet. Briefly, the slides were incubated overnight with 70% ethanol and then incubated with the luxol solution (0.1% Solvent blue 38, Sigma; 0.05% glacial acetic acid; 95% ethanol). After washes with ethanol 95% and distilled water, the slides were incubated in lithium carbonate and 70% ethanol. Then, the slides were rehyrated for 1 minut in tap water and stained with cresyl violet solution (3.1 mM, Sigma, 0.05% glacial acetic acid) for 2h, washed with distilled water, dehydrated and mounted with DPX (Fluka, Madrid, Spain). Consecutive slides were immunostained against the glial fibrillary acidic protein (GFAP). After several washes with PBS, the slides were incubated in PBS with 0.3% Triton-X-100 (Fluka) and 5% FCS (Biological Industries, Israel) for 1 h and then overnight at 4°C with a rabbit antibody against GFAP (1:1000, Dako). After washes, sections were incubated with anti-rabbit Cy3 conjugated immunoglobulin G (1:200, Jackson Immunoresearch) overnight at 4°C. Following additional washes with PBS, sections were dehydrated in ethanol and coverslipped with DPX (Fluka).

Low-power magnification (x4) images of spinal cord sections, stained with cresyl violet- luxol fast blue and GFAP-immunostained spinal cord sections were taken with the aid of a digital camera (Olympus DP50) attached to the microscope (Olympus BX-51). These images were used to measure the area of preserved spinal cord parenchyma and the area of residual white matter (i.e. normal appearing or hypomyelinated white matter). Area measurements were performed using ImageJ software and expressed as the percentage of the total area of the spinal cord section.

Motoneurons were identified by their localization in the lateral ventral horn of spinal cord sections stained with cresyl violet and were counted following a method published elsewhere (Grossman et al, 2001) by size and morphological strict criteria. Microphotographs covering the whole ventral horn were taken at 200X, and a 30 µm square grid was superimposed onto each one. Only motoneurons with diameter larger than one square, i.e. ranging from 30 to 70 (m, and with a prominent nucleolus and being polygonal in shape were counted. The mean number of motoneurons per section was calculated.

### Immunohistochemistry

Transverse sections were rehydrated and treated for antigen retrieval with 10.2 mM sodium citrate buffer (pH 6.1) during 5 minutes low heating in a microwave. Sections were then incubated at room temperature for 2 h with the following primary antibodies, rabbit anti-grp78 (Stressgen Biotechnologies, 1/200), anti-GFAP and NeuN(Chemicon, Temecula, CA, USA; 1/1000), mouse anti-gadd153 (Santa Cruz Biotechnologies, 1/200), RT97 (Hybridoma Bank, University of Iowa, USA), anti-serotonin (Diasorin, Stillwater, USA), CC1/APC (Abcam, Cambridge, UK). After washes they were incubated for 2 h at room temperature with Cy-2 or Cy-3 conjugated donkey antibodies (Jackson Immunoresearch, 1/200). After washing, the slides were counterstained with DAPI (Sigma, 1/2000), dehydrated and mounted with DPX.

### Protein extraction and Western blot

Rats were re-anesthetised and decapitated at 3 dpo for sample preparation. For protein extraction, the T8-T9 cord segment was removed and homogenized in RIPA modified buffer (50 mM Tris-HCl ph 7.5, 1 % Triton X-100, 0.5% DOC Na, 0.2 % SDS, 100 mM NaCl, 1 mM EDTA) with 10 µl/ml of Protease Inhibitor Cocktail (Sigma). After clearance, protein concentration was measured by the BCA method assay (BCA Protein Assay kit, Pierce). Equal amount of protein were loaded from each sample in 12% SDS-polyacrylamide gels. The transfer buffer was 25 mM trizma-base, 192 mM glycine, 20% (v/v) methanol, pH 8.4. The primary antibodies used were: mouse anti-β-actin (Sigma; 1/5000), rabbit anti-activating transcription factor 4 (ATF4) (Santa Cruz Biotechnologies, 1/100), rabbit anti-phosphorylated alpha subunit of eukaryotic initiation factor 2 (p-eIF2α) (Epitomics; 1/500), rabbit anti-total eIF2α (Cell Signalling Technology, 1/1000) and rabbit anti- acetylated histone-3 (acetyl-H3) (Upstate, New York; 1/500). The membrane was visualized by enhanced chemiluminiscence (ECL) method and the images analyzed with Gene Snap and Gene Tools software, and Gene Genome apparatus (Syngene).

### Statistical analysis

Functional, electrophysiological and histological measurements were performed in a blinded manner, using a code of rats and histological slices. Data are shown as the mean ± SEM. Statistical comparisons between groups were made by two-way ANOVA followed by Bonferroni post-hoc tests, or by unpaired t-tests. Differences were considered significant if p<0.05.

### RESULTS

### VPA treatment reduces CHOP accumulation after SCI

We have recently reported that SCI triggers an unbalanced UPR characterized by unchanged levels of BiP/Grp78 levels but progressive accumulation of the pro-apoptotic protein CHOP, with maximal CHOP levels found at 3 days post-injury in the weight-drop contusion model (Penas et al, 2007). In this study, we used a contusion model using an impactor device to specifically control the force (either 150 KDyn or 250 KDyn) to be apply on the T8 laminectomized spinal cord segment. We analyzed the spinal cord 8 mm-length segment that includes the lesion epicenter from sham-operated animals and from VPA treated or vehicle treated SCI animals at 3 days after operation. Western blot analyses revealed a negligible CHOP accumulation in non-injured spinal cords from sham-operated rats. In lesioned rats, BiP levels were similar to those observed in sham animals, while CHOP levels were significantly elevated as expected **(****Fig. 1a****-c).** VPA administered at 300mg/kg once per day did not produce changes on the levels of these factors over the saline administration (Fig. 1a). Thus, we increased the amount of VPA administered to 300 mg/kg every 12 h, as reported previously for a brain stroke rat model (Kim et al, 2007), and analyzed the effect using two different contusion forces of 150 and 250 KDyn. We observed that the levels of CHOP were directly proportional to the severity of the lesion; the more severe, the higher the level. Futhermore, at three days post-injury, we observed that this higher dosage of VPA was not able to alter BiP levels, but it instead significantly reduced CHOP levels (Fig. 1); effect more pronounced in the more severely (250 KDyn) injured spinal cords. Thus, we decided to focus on the 250 KDyn contusion SCI for further analyses, and treat the animals with doses of VPA every 12 h during the first week and then once daily during two more weeks. We investigated whether other targets of VPA were altered in the SCI model. VPA has been shown to work as an histone deacetylase inhibitor (Phiel et al, 2001) among other activities. Thus, we investigated the levels of acetylated histone-3 (acetyl-H3) since they might be affected by deacetylation inhibition promoted by VPA. We found that the levels of acetylated H3 increased significantly following SCI compared with spinal cords from sham animals, and that they were further increased by VPA treatment **(****Fig. 1d****).** We addressed the issue whether the effect of VPA on CHOP might be also obtained when submitted the cells to specific ER stress. Thus, we prepared *in vitro* cell cultures and stressed the cells with 3 µg/ml tunicamycin that blocks protein N-glycosilation and caused ER overload. Concomitantly, saline vehicle or different concentrations of valproate was added. Western blot analysis revealed that in either primary fibroblast cells or in the HEK cell line the CHOP accumulation promoted by tunicamycin can be attenuated with the use of 1.8 nM valproate **(****Fig. 1e, f)**. In contrast, no effect on BiP levels was observed.

In light of the marked reduction of CHOP induction observed in the animals treated with VPA, we asked whether other components of the UPR pathway upstream of CHOP were also altered. During ER stress CHOP is mainly induced via activation of the ER-localized kinase doublestranded RNA-activated protein kinase (PKR)-like ER kinase (PERK) through the downstream phosphorylation of a translation initiation factor, eukaryotic initiation factor 2α (eIF2α), and induction of a transcription factor, activation transcription factor 4 (ATF4) (Harding et al., 2000). We found significantly higher levels of ATF4 in VPA-treated injured spinal cords compared with vehicle-treated ones, but not in P-eIF2α **(****Fig. 2****)**. These results revealed that VPA exerts its action on more than one component of the UPR, upstream of CHOP-related regulation.

### Effect of VPA on hind limb locomotion after SCI

In order to assess if the molecular protective actions promoted by VPA resulted in beneficial neuroprotective effects after SCI, we evaluated two groups of SCI rats (n=6 per group) that were treated with either vehicle-saline or VPA, and followed up during 35 days postinjury. Pre-operatively, all animals showed normal hind limb movements in open-field locomotion (scored 21 points in the BBB scale, (Basso et al, 1996). After spinal cord contusion all rats developed complete hind limb paralysis. By 7 days post-surgery, the BBB scores averaged about 3-4 in the two groups of rats. Thereafter, VPA-treated animals showed higher partial recovery of locomotion than saline-treated animals, reaching significantly higher scores at 35 days (12 *vs* 8) **(****Fig. 3a****)**.

Electrophysiological studies revealed that following injury, the latency and the amplitude of the M wave elicited by stimulation of the sciatic nerve did not vary with respect to normal values **(****Fig. 3b****)**. However, the amplitude of the H wave increased in all injured rats. The mean H/M amplitude ratio was about 9% at 14 days, and increased to 20% in the control group and to 16% in the VPA group at 28 days. MEPs recorded in the tibialis anterior muscle in intact animals consisted of a single negative wave with amplitude of about 20 mV and a latency of around 6 ms. In injured rats, the MEPs were markedly decreased in amplitude and delayed in latency, indicating severe disruption of descending motor pathways **(****Fig. 3b****)**. The mean amplitudes of MEPs were slightly higher in group VPA than in group control at 14 and 28 days postlesion. Thus, VPA treatment improved overall hind limb motor function after SCI compared with saline treated controls.

### VPA promotes tissue sparing after SCI

Histologically the injured spinal cords showed an incomplete rim of residual hypomyelinated white matter, surrounding a central area of cavities and destroyed tissue at the lesion epicenter. The lesion extended to over 3 mm rostrally and caudally tapering gradually to cavities affecting central and dorsal areas of the spinal cord gray and white matter **(****Fig. 4a****)**. Measurement of the total volume of the lesion indicated also a significant effect of VPA **(****Fig. 4b****)**. VPA treatment significantly increased the area of total tissue preserved as well as that of white matter at the epicenter and adjacent section of the spinal cord **(****Fig. 4c****)**.

We also observed that the group of rats treated with VPA presented fewer lesions in the sections distal to the epicenter, with healthy-appearance ventral horn neurons with euchromatic nuclei and prominent nucleoli **(****Fig. 4d****)**. The number of NeuN neurons in this area seem to be more abundant. Thus, ventral horn motoneurons were counted on cresyl violet stained sections by size exclusion. A reduction in the length of spinal cord devoid of motoneurons was found in the VPA treated group, especially in the caudal side, although it did not reach statistical significance **(****Fig. 4e****)**.

Therefore, it can be stated that the reduction of CHOP levels by the administration of valproate comprises promoting tissue sparing.

### VPA treatment reduces myelin, oligodendrocyte and axonal loss

We analysed sections at 2 mm from the epicenter stained with cresyl violet and luxol fast blue (Klüber-Barrier staining) and observed that VPA treatment markedly reduced the extent of myelin loss after injury **(****Fig. 5a****)**. We then performed immunolabeling with CC1 antibody to count the number of oligodencrocytes presented in the sections placed at 1 and 2 mm caudal to the epicentre. We co-immunolabeled the sections with anti-GFAP to exclude false positives. We observed that VPA treatment markedly increased the proportion of oligodendrocytes presented compared to vehicle treated animals **(****Fig. 5b,c****).** We wanted to analyse any effect of VPA on other glial cells and thus we determined the total GFAP immunoreactivity in astrocytes across the sections. In this regard, no significant alterations were observed along the damaged spinal cord in treated rats at 35 dpo **(****Fig. 5d****)**.

We also analyzed the presence of axons in the same areas by immunohistochemical staining with anti-neurofilament antibody RT97. As shown in figure **6a** and **b**, samples from rats treated with VPA showed an increased density of axonal profiles from descending/ascending axonal spinal pathways at levels both rostral and caudal to the lesion as well as the number of serotonin (5-HT)-positive axons.

Therefore, it can be stated that the reduction of CHOP levels by the administration of valproate comprises reducing demyelination, promoting oligodendrocyte survival and reducing axonal loss.

### Discussion of results

With this study, the present inventors have proved that treatment with valproate at therapeutically effective doses promotes neuroprotection and functional recovery in a rat model of SCI by reducing lesion volume, demyelination and axonal loss. The molecular mechanisms by which VPA exerts its neuroprotective action in this model include the prevention of ER stress associated pro-apoptotic signalling mediated by CHOP.

From a mechanistic point of view, UPR triggered by ER stress is an inter-organelle signalling from the ER to the nucleus to maintain the homeostasis of protein folding altered by different kinds of cell damage, such as glucose deprivation, hypoxia and alteration in the calcium homeostasis (Kaufman, 1999). Excessive or long-term accumulation of unfolded proteins in the ER results in apoptosis of the cells. A mediator of ER stress-induced cell death is CHOP (Wang et al., 1996) (Zinszner et al., 1998); (Oyadomari et al., 2001), (Murphy et al., 2001). The expression of CHOP is tightly regulated at post-transcriptional level, but after ER stress it can be accumulated and moved into the nucleus where it exerts its pro-apototic action. This action is still far from clear but it is known that once in the nucleus, CHOP may act as a transcription factor or it may impede the transcription of classical pro-survival genes such as Bcl-2. Interestingly, CHOP-deficient mice have been shown to be resistant to apoptosis in various disease models (Tajiri et al., 2004), (Endo et al., 2006), (Tamaki et al., 2008) and to exhibit significantly less programmed cell death when challenged with agents that perturb ER function (Zinszner et al., 1998). We have recently reported that CHOP accumulates and it is predominantly present in the nucleus of neurons and oligodendrocytes, both prone to die early after SCI (Penas et al., 2007). Our present results further prove that counteracting of ER stress by treatment with VPA offers partial neuroprotection. In addition to the increased amount of spared cord parenchima around the contusion injury, there was also improved neuronal survival, as exemplified by our counts of spinal motoneurons. Oligodendroglial death that occurs immediately after injury or during secondary degeneration results in demyelination or loss of myelin sheaths. Axonal demyelination is thought to be a major contributor to the loss of function following SCI due to impaired impulse conduction and attenuated action potential propagation, which may be a result of the reorganization of ion channels. Demyelination is increasingly being considered as a therapeutic target for SCI (Cao et al., 2005); (Karimi-Abdolrezaee et al., 2006). Thus, it is feasible that CHOP reduction promoted by VPA treatment might prevent oligodendroglial death and hence demyelination after SCI. The importance of CHOP in demyelinating pathologies in either peripheral or central nervous system has been pointed out recently. For instance, Chop ablation in a Charcot-Marie-Tooth 1B mouse model completely rescues the motor deficit and reduces active demyelination in the peripheral nerves (Pennuto et al., 2008). Thus, the reduction of demyelination observed after SCI following VPA treatment might be related to the decrease in CHOP protein levels.

According to the observed results, the present inventors have envisaged several possibilities about how VPA modulates CHOP levels. During ER stress, CHOP protein is regulated through modulation of its degradation by the proteasome (Hattori et al., 2003). The N-terminal portion required for CHOP degradation is the same region required for its interaction with p300, a transcriptional coactivator of CHOP and other proteins with acetylase and ubiquitin ligase activities (Ohoka et al., 2007). Curiously, VPA affects intracellular trafficking of p300 (Chen et al., 2007), shuttling it out from the nucleus (Chen et al., 2007) and once in the cytoplasm, VPA also induces p300 degradation through the proteasome (Chen et al., 2005). Thus, one possibility is that CHOP might be sequestered by its interaction with p300 and follows it in its journey towards the cytoplasm promoted by VPA, where it may be degraded. VPA interfering on intracellular trafficking of some proteins, like p300 and possibly CHOP might explain the reduction in CHOP accumulation after SCI.

Intriguingly, the accumulation of ATF4 we observed after SCI in VPA-treated animals can be explained in a similar way, because p300 is also an interaction partner of ATF4. The interactive complex stimulates ATF4-driven transcription via p300 histone acetyltransferase domain ((Stiehl et al., 2007). In contrast to CHOP, ATF4 is susceptible to acetylation mediated by p300 and its acetylated status is affected by TSA, a specific inhibitor of histone deacetylases ((Stiehl et al., 2007). Thus, the inhibition of cellular deacetylases might lead to a longer half-life of ATF4, similarly to what has been reported for other proteins like p53, since acetylation by p300 is required for ubiquitination and consequent degradation (Ito et al., 2002) (Brooks and Gu, 2003). VPA acts as an inhibitor of histone deacetylase enzyme activity (Phiel et al., 2001) among several other targets(Chen et al., 2006), (Gobbi and Janiri, 2006); (Loscher, 1999). We have shown this activity in the SCI model and so it might be related to the accumulation of ATF4 observed.

### REFERENCES

Basso, D. M., Beattie, M. S. and Bresnahan, J. C., 1996. Graded histological and locomotor outcomes after spinal cord contusion using the NYU weight-drop device versus transection. Exp Neurol. 139, 244-256.
Beattie, M. S., Harrington, A. W., Lee, R., Kim, J. Y., Boyce, S. L., Longo, F. M., Bresnahan, J. C., Hempstead, B. L. and Yoon, S. O., 2002. ProNGF induces p75-mediated death of oligodendrocytes following spinal cord injury. Neuron. 36, 375-386.
Bertolotti, A., Zhang, Y., Hendershot, L. M., Harding, H. P. and Ron, D., 2000. Dynamic interaction of BiP and ER stress transducers in the unfolded-protein response. Nat Cell Biol. 2, 326-332.
Brooks, C. L. and Gu, W., 2003. Ubiquitination, phosphorylation and acetylation: the molecular basis for p53 regulation. Curr Opin Cell Biol. 15, 164-171.
Cao, Q., Xu, X. M., Devries, W. H., Enzmann, G. U., Ping, P., Tsoulfas, P., Wood, P. M., Bunge, M. B. and Whittemore, S. R., 2005. Functional recovery in traumatic spinal cord injury after transplantation of multineurotrophin-expressing glial-restricted precursor cells. J Neurosci. 25, 6947-6957.
Collazos-Castro, J. E., Soto, V. M., Gutierrez-Davila, M. and Nieto-Sampedro, M., 2005. Motoneuron loss associated with chronic locomotion impairments after spinal cord contusion in the rat. J Neurotrauma. 22, 544-558.
Chen, J., Halappanavar, S., Th' ng, J. P. and Li, Q., 2007a. Ubiquitin-dependent distribution of the transcriptional coactivator p300 in cytoplasmic inclusion bodies. Epigenetics. 2, 92-99.
Chen, J., St-Germain, J. R. and Li, Q., 2005. B56 regulatory subunit of protein phosphatase 2A mediates valproic acid-induced p300 degradation. Mol Cell Biol. 25, 525-532.
Chen, P. S., Peng, G. S., Li, G., Yang, S., Wu, X., Wang, C. C., Wilson, B., Lu, R. B., Gean, P. W., Chuang, D. M. and Hong, J. S., 2006. Valproate protects dopaminergic neurons in midbrain neuron/glia cultures by stimulating the release of neurotrophic factors from astrocytes. Mol Psychiatry. 11, 1116-1125.
Emery, E., Aldana, P., Bunge, M. B., Puckett, W., Srinivasan, A., Keane, R. W., Bethea, J. and Levi, A. D., 1998. Apoptosis after traumatic human spinal cord injury. J Neurosurg. 89, 911-920.
Endo, M., Mori, M., Akira, S. and Gotoh, T., 2006. C/EBP homologous protein (CHOP) is crucial for the induction of caspase-11 and the pathogenesis of lipopolysaccharide-induced inflammation. J Immunol. 176, 6245-6253.
Garcia-Alias, G., Valero-Cabre, A., Lopez-Vales, R., Fores, J., Verdu, E. and Navarro, X., 2006. Differential motor and electrophysiological outcome in rats with mid-thoracic or high lumbar incomplete spinal cord injuries. Brain Res. 1108, 195-204.
Garcia-Alias, G., Verdu, E., Fores, J., Lopez-Vales, R. and Navarro, X., 2003. Functional and electrophysiological characterization of photochemical graded spinal cord injury in the rat. J Neurotrauma. 20, 501-510.
Gobbi, G. and Janiri, L., 2006. Sodium- and magnesium-valproate in vivo modulate glutamatergic and GABAergic synapses in the medial prefrontal cortex. Psychopharmacology (Berl). 185, 255-262.
Harding, H. P., Novoa, I., Zhang, Y., Zeng, H., Wek, R., Schapira, M. and Ron, D., 2000. Regulated translation initiation controls stress-induced gene expression in mammalian cells. Mol Cell. 6, 1099-1108.
Hattori, T., Ohoka, N., Inoue, Y., Hayashi, H. and Onozaki, K., 2003. C/EBP family transcription factors are degraded by the proteasome but stabilized by forming dimer. Oncogene. 22, 1273-1280.
Ito, A., Kawaguchi, Y., Lai, C. H., Kovacs, J. J., Higashimoto, Y., Appella, E. and Yao, T. P., 2002. MDM2-HDAC1-mediated deacetylation of p53 is required for its degradation. Embo J. 21, 6236-6245.
Karimi-Abdolrezaee, S., Eftekharpour, E., Wang, J., Morshead, C. M. and Fehlings, M. G., 2006. Delayed transplantation of adult neural precursor cells promotes remyelination and functional neurological recovery after spinal cord injury. J Neurosci. 26, 3377-3389.
Kaufman, R. J., 1999. Stress signaling from the lumen of the endoplasmic reticulum: coordination of gene transcriptional and translational controls. Genes Dev. 13, 1211-1233.
Kim, H. J., Rowe, M., Ren, M., Hong, J. S., Chen, P. S. and Chuang, D. M., 2007. Histone deacetylase inhibitors exhibit anti-inflammatory and neuroprotective effects in a rat permanent ischemic model of stroke: multiple mechanisms of action. J Pharmacol Exp Ther. 321, 892-901.
Loscher, W., 1999. Valproate: a reappraisal of its pharmacodynamic properties and mechanisms of action. Prog Neurobiol. 58, 31-59.
Mamady, H. and Storey, K. B., 2008. Coping with the stress: Expression of ATF4, ATF6, and downstream targets in organs of hibernating ground squirrels. Arch Biochem Biophys.
Murphy, T. C., Woods, N. R. and Dickson, A. J., 2001. Expression of the transcription factor GADD153 is an indicator of apoptosis for recombinant chinese hamster ovary (CHO) cells. Biotechnol Bioeng. 75, 621-629.
Ohoka, N., Hattori, T., Kitagawa, M., Onozaki, K. and Hayashi, H., 2007. Critical and functional regulation of CHOP (C/EBP homologous protein) through the N-terminal portion. J Biol Chem. 282, 35687-35694.
Oyadomari, S., Koizumi, A., Takeda, K., Gotoh, T., Akira, S., Araki, E. and Mori, M., 2002. Targeted disruption of the Chop gene delays endoplasmic reticulum stress-mediated diabetes. J Clin Invest. 109, 525-532.
Oyadomari, S., Takeda, K., Takiguchi, M., Gotoh, T., Matsumoto, M., Wada, I., Akira, S., Araki, E. and Mori, M., 2001. Nitric oxide-induced apoptosis in pancreatic beta cells is mediated by the endoplasmic reticulum stress pathway. Proc Natl Acad Sci U S A. 98, 10845-10850. Epub 12001 Aug 10828.
Penas, C., Guzman, M. S., Verdu, E., Fores, J., Navarro, X. and Casas, C., 2007. Spinal cord injury induces endoplasmic reticulum stress with different cell-type dependent response. J Neurochem. 102, 1242-1255.
Pennuto, M., Tinelli, E., Malaguti, M., Del Carro, U., D'Antonio, M., Ron, D., Quattrini, A., Feltri, M. L. and Wrabetz, L., 2008. Ablation of the UPR-mediator CHOP restores motor function and reduces demyelination in Charcot-Marie-Tooth 1B mice. Neuron. 57, 393-405.
Phiel, C. J., Zhang, F., Huang, E. Y., Guenther, M. G., Lazar, M. A. and Klein, P. S., 2001. Histone deacetylase is a direct target of valproic acid, a potent anticonvulsant, mood stabilizer, and teratogen. J Biol Chem. 276, 36734-36741.
Stiehl, D. P., Fath, D. M., Liang, D., Jiang, Y. and Sang, N., 2007. Histone deacetylase inhibitors synergize p300 autoacetylation that regulates its transactivation activity and complex formation. Cancer Res. 67, 2256-2264.
Tajiri, S., Oyadomari, S., Yano, S., Morioka, M., Gotoh, T., Hamada, J. I., Ushio, Y. and Mori, M., 2004. Ischemia-induced neuronal cell death is mediated by the endoplasmic reticulum stress pathway involving CHOP. Cell Death Differ. 11, 403-415.
Tamaki, N., Hatano, E., Taura, K., Tada, M., Kodama, Y., Nitta, T., Iwaisako, K., Seo, S., Nakajima, A., Ikai, I. and Uemoto, S., 2008. CHOP deficiency attenuates cholestasis-induced liver fibrosis by reduction of hepatocyte injury. Am J Physiol Gastrointest Liver Physiol. 294, G498-505.
Tator, C. H. and Fehlings, M. G., 1991. Review of the secondary injury theory of acute spinal cord trauma with emphasis on vascular mechanisms. J Neurosurg. 75, 15-26.
Valero-Cabre, A., Fores, J. and Navarro, X., 2004. Reorganization of reflex responses mediated by different afferent sensory fibers after spinal cord transection. J Neurophysiol. 91, 2838-2848.
Wang, X. Z., Lawson, B., Brewer, J. W., Zinszner, H., Sanjay, A., Mi, L. J., Boorstein, R., Kreibich, G., Hendershot, L. M. and Ron, D., 1996. Signals from the stressed endoplasmic reticulum induce C/EBP-homologous protein (CHOP/GADD153). Mol Cell Biol. 16, 4273-4280.
Zinszner, H., Kuroda, M., Wang, X., Batchvarova, N., Lightfoot, R. T., Remotti, H., Stevens, J. L. and Ron, D., 1998. CHOP is implicated in programmed cell death in response to impaired function of the endoplasmic reticulum. Genes Dev. 12, 982-995.

## Claims

1. The use of valproate in the manufacture of a drug for reducing C/EBP homologous transcription factor (CHOP) levels in a mammal.

2. The use according to claim 1, wherein the mammal has spinal cord injury (SCI).

3. The use according to claim 2, wherein the SCI is a traumatic SCI.

4. The use according to any one of claims 1 to 3, wherein the valproate is administered in a dose regimen comprising the initial administration of 1 to 15 mg/kg total body weight of valproate after at least 3 hours post-injury and a daily administration of 20 to 60 mg/kg total body weight of valproate from 12 hours after the first dose and for at least 21 days.

5. The use according to claim 4, wherein the daily administration is from 30 to 50 mg/kg total body weight of valproate.

6. The use according to claim 4 or 5, wherein the valproate is administered along with other active ingredients, stem cells or cell types selected from the group comprising Schwann cells and olfactory ensheating glial cells.

7. The use according to any one of claims 1 to 6, wherein the reduction of CHOP levels comprises promoting tissue sparing.

8. The use according to any one of claims 1 to 7, wherein the reduction of CHOP levels comprises reducing demyelination, promoting oligodendrocyte survival and reducing axonal loss

9. The use of valproate in the manufacture of a drug for treating SCI in a mammal.

10. The use according to claim 9, wherein the SCI is a traumatic SCI.

11. Valproate for use as a neuroprotective agent in a mammal having traumatic SCI.

12. Valproate for use in reducing CHOP levels in a mammal.

13. The method for reducing CHOP levels in a mammal comprising the administration of a therapeutically effective amount of valproate.

14. Valproate according to claim 12 or method according to claim 13, wherein the mammal has SCI.

15. Valproate according to claim 14 or method according to claim 14, wherein the SCI is a traumatic SCI.

16. The use according to any one of claims 1 to 10; valproate according to any one of claims 11, 12, 14 or 15; or method according to any one of claims 13, 14 or 15, wherein the mammal is a human being.
